(19)

Europäisches
Patentamt
European
Patent Office
Office européen
des brevets

(11) **EP 4 273 103 A1**

(12) **EUROPEAN PATENT APPLICATION**
published in accordance with Art. 153(4) EPC

(43) Date of publication:
**08.11.2023 Bulletin 2023/45**

(21) Application number: **22745770.2**

(22) Date of filing: **21.01.2022**

(51) International Patent Classification (IPC):
$C02F\ 5/00^{(2023.01)}$       $G05B\ 23/02^{(2006.01)}$
$C02F\ 1/00^{(2023.01)}$       $G06Q\ 50/06^{(2012.01)}$
$G01N\ 33/18^{(2006.01)}$

(52) Cooperative Patent Classification (CPC):
**C02F 1/00; C02F 5/00; G01N 33/18; G05B 23/02;
G06Q 50/06**

(86) International application number:
**PCT/JP2022/002303**

(87) International publication number:
**WO 2022/163549 (04.08.2022 Gazette 2022/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.01.2021   JP 2021013920**

(71) Applicant: **Kurita Water Industries Ltd.
Tokyo 164-0001 (JP)**

(72) Inventors:
• **OBUNAI, Kishin
  Tokyo 164-0001 (JP)**
• **TOYOOKA, Yasuhiro
  Tokyo 164-0001 (JP)**
• **WADA, Satoshi
  Tokyo 164-0001 (JP)**
• **HARADA, Kaname
  Tokyo 164-0001 (JP)**
• **TAGUCHI, Chigusa
  Tokyo 164-0001 (JP)**
• **FUTAKI, Sakae
  Tokyo 164-0001 (JP)**

(74) Representative: **TBK
Bavariaring 4-6
80336 München (DE)**

(54) **ESTIMATION DEVICE, PREDICTION DEVICE, CONTROL DEVICE, ESTIMATION SYSTEM, PREDICTION SYSTEM, CONTROL SYSTEM, ESTIMATION PROGRAM, PREDICTION PROGRAM, CONTROL PROGRAM, ESTIMATION METHOD, PREDICTION METHOD, AND CONTROL METHOD**

(57) To provide an estimation device, a prediction device, a control device, an estimation system, a prediction system, a control system, an estimation program, a prediction program, a control program, an estimation method, a prediction method, and a control method which are capable of estimating an index value to act as a measure of generation of scale from water quality information being readily measurable during operation. An aspect of the present invention provides an estimation device which estimates a scaling index value of a water system. The estimation device includes: a water quality information acquiring unit; a relational model information acquiring unit; and an estimating unit. The water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system. The relational model information acquiring unit acquires relational model information including a relational model indicating a relationship between a scaling index value which is calculated from a function created in advance of concentrations of ions constituting scale that may be formed in the water system and the water quality parameters. The estimating unit calculates an estimated scaling index value based on the water quality information and the relational model information.

EP 4 273 103 A1

# Fig. 1

**Description**

Technical Field

The present invention relates to an estimation device, a prediction device, a control device, an estimation system, a prediction system, a control system, an estimation program, a prediction program, a control program, an estimation method, a prediction method, and a control method.

Background Art

In a water system, operational troubles may occur, such as various ions contained in the water being precipitated as scale and deposited in pipes or the like. In consideration thereof, generation of scale is detected and various chemicals for inhibiting scale are added.

The scale is generated when a product of concentrations of the respective ions that make up the scale exceeds a solubility product of the ions. Utilizing this causality, for example, NPL1 and NPL2 describe using a logarithmic value of a ratio of a product of valence powers of concentrations of the respective ions relative to the solubility product of the respective ions as a saturation index in order to predict whether scale is to be generated or not. Using such an index enables the generation of scale to be accurately detected.

Citation List

Non Patent Literature

NPL1: Kenji Kowata, JAPAN TAPPI JOURNAL, Vol. 57, No. 7, Page 70, 2003
NPL2: Kenji Kowata, Japanese Journal of Paper Technology, Vol. 44, No. 7, Page 29, 2001

Summary of Invention

Technical Problem

In order to suppress the generation of scale more efficiently, desirably, an index value to act as a measure of scale generation is promptly calculated and various chemicals for inhibiting scale are added in accordance with the index value in a timely manner. However, with the methods of calculating a saturation index according to NPL1 and NPL2, measuring respective ion concentrations of cations and anions that make up scale often take time and a saturation index cannot always be calculated in an expeditious manner.

In consideration of the circumstances described above, an object of the present invention is to provide an estimation device, a prediction device, a control device, an estimation system, a prediction system, a control system, an estimation program, a prediction program, a control program, an estimation method, a prediction method, and a control method which are capable of estimating an index value to act as a measure of generation of scale from water quality information being readily measurable during operation.

Solution to Problem

An aspect of the present invention provides an estimation device which estimates a scaling index value of a water system. The estimation device includes: a water quality information acquiring unit; a relational model information acquiring unit; and an estimating unit. The water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system. The relational model information acquiring unit acquires relational model information including a relational model indicating a relationship between a scaling index value which is calculated from a function created in advance of concentrations of ions constituting scale that may be formed in the water system and the water quality parameters. The estimating unit calculates an estimated scaling index value based on the water quality information and the relational model information.

The present invention may be provided in each of the aspects described below.

The estimation device described above, wherein the relational model is a model obtained by a regression analysis of the scaling index value and a scaling index estimation function that is a function of the water quality parameters.

The estimation device described above, wherein the water quality parameters include at least electrical conductivity.

A prediction device which predicts scale generation in a water system, the prediction device including: a water quality information acquiring unit; a relational model information acquiring unit; an estimating unit; and a predicting unit, wherein the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system, the relational model information acquiring unit acquires relational model information including a relational model indicating a relationship between a scaling index value which is calculated from a function created in advance of concentrations of ions constituting scale that may be formed in the water system and the water quality parameters, the estimating unit calculates an estimated scaling index value based on the water quality information and the relational model information, and the predicting unit predicts, based on the estimated scaling index value, a presence or absence or a possibility of generation of scale.

A control device which controls addition of a scale inhibitor with respect to a water system, the control device including: a water quality information acquiring unit; a relational model information acquiring unit; an estimating unit; a scale inhibitor addition model information acquiring unit; and an additive amount determining unit, wherein the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system, the relational model information acquiring unit acquires relational model information including a relational model indicating a relationship between a scaling index value which is calculated from a function created in advance of concentrations of ions constituting scale that may be formed in the water system and the water quality parameters, the estimating unit calculates an estimated scaling index value based on the water quality information and the relational model information, the scale inhibitor addition model information acquiring unit acquires scale inhibitor addition model information including a scale inhibitor addition model which has been created in advance and which indicates a relationship between the estimated scaling index value and an additive amount of a scale inhibitor, and the additive amount determining unit determines the additive amount of the scale inhibitor based on the estimated scaling index value and the scale inhibitor addition model information.

An estimation system which estimates a scaling index value of a water system, the estimation system including: a water quality information acquiring unit; a relational model information acquiring unit; and an estimating unit, wherein the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system, the relational model information acquiring unit acquires relational model information including a relational model indicating a relationship between a scaling index value which is calculated from a function created in advance of concentrations of ions constituting scale that may be formed in the water system and the water quality parameters, and the estimating unit calculates an estimated scaling index value based on the water quality information and the relational model information.

A prediction system which predicts scale generation in a water system, the prediction system including: a water quality information acquiring unit; a relational model information acquiring unit; an estimating unit; and a predicting unit, wherein the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system, the relational model information acquiring unit acquires relational model information including a relational model indicating a relationship between a scaling index value which is calculated from a function created in advance of concentrations of ions constituting scale that may be formed in the water system and the water quality parameters, the estimating unit calculates an estimated scaling index value based on the water quality information and the relational model information, and the predicting unit predicts, based on the estimated scaling index value, a presence or absence or a possibility of generation of scale.

A control system which controls addition of a scale inhibitor with respect to a water system, the control system including: a water quality information acquiring unit; a relational model information acquiring unit; an estimating unit; a scale inhibitor addition model information acquiring unit; and an additive amount determining unit, wherein the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system, the relational model information acquiring unit acquires relational model information including a relational model indicating a relationship between a scaling index value which is calculated from a function created in advance of concentrations of ions constituting scale that may be formed in the water system and the water quality parameters, the estimating unit calculates an estimated scaling index value based on the water quality information and the relational model information, the scale inhibitor addition model information acquiring unit acquires scale inhibitor addition model information including a scale inhibitor addition model which has been created in advance and which indicates a relationship between the estimated scaling index value and an additive amount of a scale inhibitor, and the additive amount determining unit determines the additive amount of the scale inhibitor based on the estimated scaling index value and the scale inhibitor addition model information.

An estimation program for estimating a scaling index value of a water system, the estimation program causing a computer to function as: a water quality information acquiring unit; a relational model information acquiring unit; and an estimating unit, wherein the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system, the relational model information acquiring unit acquires relational model information including a relational model indicating a relationship between a scaling index value which is calculated from a function created in advance of concentrations of ions constituting scale that may be formed in the water system and the water quality parameters, and the estimating unit calculates an estimated scaling index value based on the water quality information and the relational model information.

A prediction program for predicting scale generation in a water system, the prediction program causing a computer to function as: a water quality information acquiring unit; a relational model information acquiring unit; an estimating unit; and a predicting unit, wherein the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system, the relational model information acquiring unit acquires relational model information including a relational model indicating a relationship between a scaling index value which is calculated from a function created in advance of concentrations of ions constituting scale that may be formed in the water system and the water quality parameters, the estimating unit calculates an estimated scaling index value based on the water quality information and the relational model information, and the predicting unit predicts, based on the estimated scaling index value, a presence or absence or a possibility of generation of scale.

A control program for controlling addition of a scale inhibitor with respect to a water system, the control program causing a computer to function as: a water quality information acquiring unit; a relational model information acquiring unit; an estimating unit; a scale inhibitor addition model information acquiring unit; and an additive amount determining unit, wherein the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system, the relational model information acquiring unit acquires relational model information including a relational model indicating a relationship between a scaling index value which is calculated from a function created in advance of concentrations of ions constituting scale that may be formed in the water system and the water quality parameters, the estimating unit calculates an estimated scaling index value based on the water quality information and the relational model information, the scale inhibitor addition model information acquiring unit acquires scale inhibitor addition model information including a scale inhibitor addition model which has been created in advance and which indicates a relationship between the estimated scaling index value and an additive amount of a scale inhibitor, and the additive amount determining unit determines the additive amount of the scale inhibitor based on the estimated scaling index value and the scale inhibitor addition model information.

An estimation method of a scaling index value of a water system, the estimation method including: a water quality information acquiring step; a relational model information acquiring step; and an estimating step, wherein in the water quality information acquiring step, water quality information including two or more water quality parameters selected from the group consisting of at least pH, a cationic demand, a residual chlorine concentration, temperature, electrical conductivity, an amount of floating suspended solids, turbidity, and chromaticity of the water system is acquired, in the relational model information acquiring step, relational model information including a relational model indicating a relationship between a scaling index value which is calculated from a function created in advance of concentrations of ions constituting scale that may be formed in the water system and the water quality parameters is acquired, and in the estimating step, an estimated scaling index value is calculated based on the water quality information and the relational model information.

A prediction method of scale generation in a water system, the prediction method including: a water quality information acquiring step; a relational model information acquiring step; an estimating step; and a predicting step, wherein in the water quality information acquiring step, water quality information including two or more water quality parameters selected from the group consisting of at least pH, a cationic demand, a residual chlorine concentration, temperature, electrical conductivity, an amount of floating suspended solids, turbidity, and chromaticity of the water system is acquired, in the relational model information acquiring step, relational model information including a relational model indicating a relationship between a scaling index value which is calculated from a function created in advance of concentrations of ions constituting scale that may be formed in the water system and the water quality parameters is acquired, in the estimating step, an estimated scaling index value is calculated based on the water quality information and the relational model information, and in the predicting step, a presence or absence or a possibility of generation of scale is predicted based on the estimated scaling index value.

A control method of addition of a scale inhibitor with respect to a water system, the control method including: a water quality information acquiring step; a relational model information acquiring step; an estimating step; a scale inhibitor addition model information acquiring step; and an additive amount determining step, wherein in the water quality information acquiring step, water quality information including two or more water quality parameters selected from the group consisting of at least pH, a cationic demand, a residual chlorine concentration, temperature, electrical conductivity, an amount of floating suspended solids, turbidity, and chromaticity of the water system is acquired, in the relational model information acquiring step, relational model information including a relational model indicating a relationship between a scaling index value which is calculated from a function created in advance of concentrations of ions constituting scale that may be formed in the water system and the water quality parameters is acquired, in the estimating step, an estimated scaling index value is calculated based on the water quality information and the relational model information, in the scale inhibitor addition model information acquiring step, scale inhibitor addition model information including a scale inhibitor addition model which has been created in advance and which indicates a relationship between the estimated scaling index value and an additive amount of a scale inhibitor is acquired, and in the additive amount determining step, an additive amount of the scale inhibitor is determined based on the estimated scaling index value and the scale inhibitor addition model information.

It is needless to say that the present invention is not limited to the above.

According to the present invention, an index value to act as a measure of generation of scale can be estimated from water quality information that is readily measurable during operation.

Brief Description of Drawings

[Fig. 1] Fig. 1 is a schematic diagram of a prediction system according to a present embodiment.

[Fig. 2] Fig. 2 is a schematic view showing a functional configuration of a prediction device according to the present embodiment.

[Fig. 3] Fig. 3 is a schematic diagram showing a hardware configuration of the prediction device according to the present embodiment.

[Fig. 4] Fig. 4 is a flowchart of a prediction method according to the present embodiment.

[Fig. 5] Fig. 5 shows plots of scaling index values against estimated scaling index values of a total of 19 data sets according to example 1.

[Fig. 6] Fig. 6 shows plots of scaling index values against estimated scaling index values of a total of 18 data sets according to example 2.

Description of Embodiment

Hereinafter, an embodiment of the present invention will be described with reference to the drawings. Various constituent elements shown in the embodiment described below can be combined with each other.

A program for realizing software according to the present embodiment may be provided as a non-transitory computer-readable medium, provided so as to be downloadable from an external server, or provided so as to be run on an external computer such that functions thereof are realized on a client terminal (so-called cloud computing).

In addition, a "unit" as referred to in the present embodiment may include, for example, a combination of hardware resources implemented by a circuit in a broad sense and information processing by software which may be specifically realized by such hardware resources. Furthermore, while various kinds of information are handled in the present embodiment, for example, such information are to be represented by a physical value of a signal value representing a voltage or a current, a level of a signal value as an aggregate of binary bits constituted of 0 or 1, or a quantum superposition (a so-called quantum bit), and communication and calculations can be executed on the circuit in a broad sense.

Moreover, the circuit in a broad sense is a circuit which is realized by at least appropriately combining a circuit, circuitry, a processor, a memory, and the like. In other words, the circuit in a broad sense includes an application specific integrated circuit (ASIC), a programmable logic device (for example, a simple programmable logic device (SPLD), a complex programmable logic device (CPLD), or a field programmable gate array (FPGA)), and the like.

<Prediction system>

A prediction system according to the present embodiment is a prediction system which predicts scale generation in a water system. Specifically, the prediction system includes a water quality information acquiring unit, a relational model information acquiring unit, an estimating unit, and a predicting unit. Among these units, the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system. The relational model information acquiring unit acquires relational model information including a relational model indicating a relationship between a scaling index value which is calculated from a function created in advance of concentrations of ions constituting scale that may be formed in the water system and the water quality parameters. The estimating unit calculates an estimated scaling index value based on the water quality information and the relational model information. The predicting unit predicts, based on the estimated scaling index value, a presence or absence or a possibility of generation of scale.

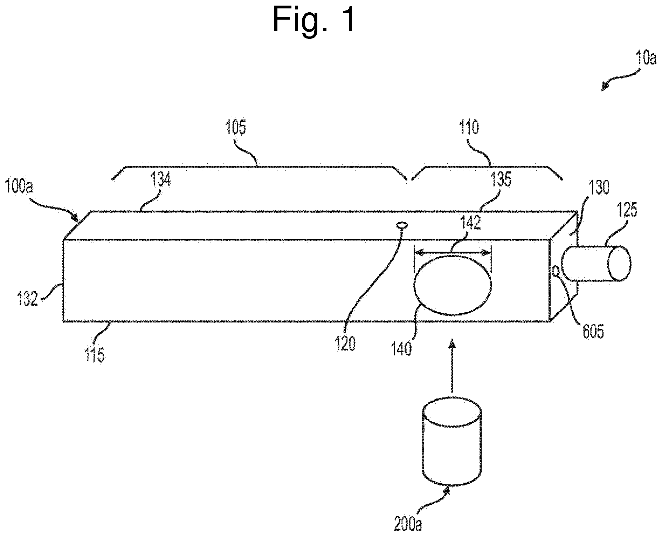

In addition, although not essential constituent elements, the prediction system according to the present embodiment may include one of or two or more of a relational model creating unit, a predictive model creating unit, a predictive model information acquiring unit, a scale inhibitor addition model creating unit, a scale inhibitor addition model information acquiring unit, an additive amount determining unit, an output unit, and a scale inhibitor adding unit. Note that a prediction system including all of these units will be mainly explained with reference to Fig. 1 to be described below.

In this case, "scale" is a general term for metallic salts that are precipitated and deposited in water systems. Specifically, scale includes, but is not particularly limited to, calcium oxalate, calcium carbonate, calcium sulfate, calcium phosphate, and barium sulfate.

A scaling index value is a numerical value which can be used as an index value of generation of scale and which is not particularly limited as long as the numerical value is calculated from a function of concentration of ions constituting scale that can be formed in the water system. For example, a saturation index SI can be used as the function. Specifically, the saturation index SI is a logarithmic value of a ratio of a product of valence powers of concentrations of respective ions relative to a solubility product of the respective ions. NPL1 describes the saturation index SI of calcium oxalate and NPL2 describes the saturation index SI of calcium carbonate. Specifically, a general formula of the saturation index SI is represented by equation (1) below, where A denotes a cation, B denotes an anion, +n denotes a valence of the cation A, -m denotes a valence of the anion B (n and m each independently representing a positive integer), and $K_{sp}$ denotes a solubility product of the cation A and the anion B.

$$SI = log \frac{[A^{n+}]^m [B^{m-}]^n}{K_{sp}} \quad \cdots (1)$$

While equation (1) represents an equation featuring one species of the cation A and one species of the anion B, the numbers of cations and anions are dependent on the type of scale generated and are not limited to one species.

As described above, a function for obtaining a scaling index value is not particularly limited to equation (1). Examples of scaling index values other than equation (1) may include a scaling index value that does not take the logarithmic value of equation (1) (inside the log symbol) (equation (2)), a reciprocal thereof (equation (3)), a reciprocal of the inside of the logarithm in equation (1) (equation (4)), scaling index values obtained by exponentiating equations (1) to (4), and scaling index values obtained by addition, subtraction, multiplication, or division of a constant.

$$SI' = \frac{[A^{n+}]^m [B^{m-}]^n}{K_{sp}} \quad \cdots (2)$$

$$SI'' = \frac{K_{sp}}{[A^{n+}]^m [B^{m-}]^n} \quad \cdots (3)$$

$$SI''' = log \frac{K_{sp}}{[A^{n+}]^m [B^{m-}]^n} \quad \cdots (4)$$

While the function for obtaining a scaling index value may or may not include the solubility product $K_{sp}$, the

function preferably includes the solubility product $K_{sp}$. In addition, the function for obtaining a scaling index value preferably includes at least one or more species of a cation or an anion and more preferably includes all species of cations and anions. In one embodiment, the function for obtaining a scaling index value is preferably a function which includes all species of cations and anions and which represents a ratio between a product of concentrations of the ion species and a solubility product of the ions (equation (2), equation (3)).

[Functional configuration of prediction system]

Fig. 1 is a schematic diagram of a prediction system according to the present embodiment. A prediction system 1 includes a prediction device 2, an output device 3, a water quality information measurement device 4, and a scale inhibitor addition device 5.

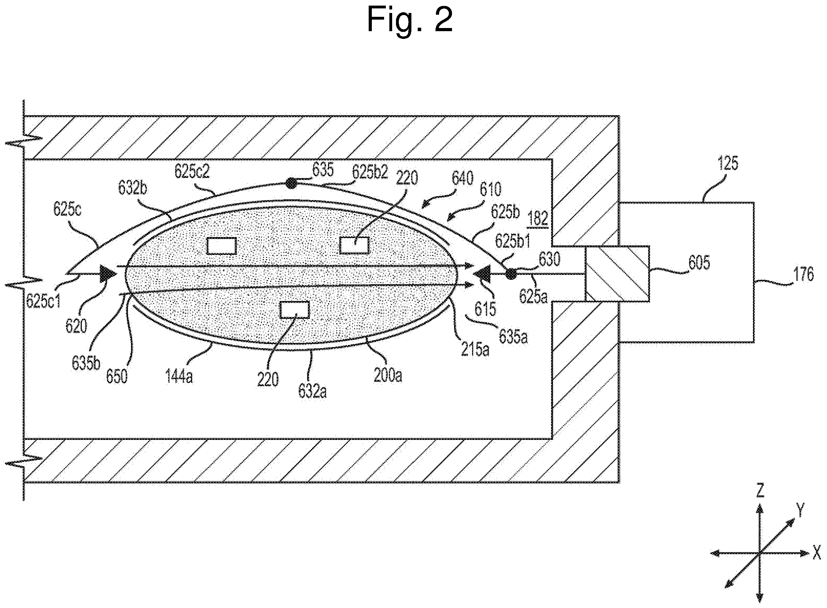

Among these components, the prediction device 2 controls information processing for prediction in the prediction system 1. Fig. 2 is a schematic view showing a functional configuration of the prediction device according to the present embodiment. As shown in Fig. 2, the prediction device 2 according to the present embodiment includes a water quality information acquiring unit 20, a relational model information acquiring unit 21, an estimating unit 22, and a predicting unit 23. In addition, the prediction device 2 according to the present embodiment further includes a relational model creating unit 24, a predictive model creating unit 25, a predictive model information acquiring unit 26, a scale inhibitor addition model creating unit 27, a scale inhibitor addition model information acquiring unit 28, and an additive amount determining unit 29. While the respective units are described herein as being included inside a single device, alternatively, each unit may be included as a separate device. Furthermore, while the output device 3 is an example of the output unit, the water quality information measurement device 4 is an example of the water quality information measuring unit, and the scale inhibitor addition device 5 is an example of the scale inhibitor adding unit, a description will be hereinafter given without particularly distinguishing the devices from the units.

[Functions of prediction system]

Hereinafter, a function of each unit of the prediction system 1 will be described in specific terms.

[Water quality information acquiring unit]

The water quality information acquiring unit 20 acquires water quality information including two or more water quality parameters selected from the group consisting of at least pH, a cationic demand, a residual chlorine concentration, temperature, electrical conductivity, an amount of floating suspended solids, turbidity, and chromaticity of a water system.

The pH, the cationic demand, the residual chlorine concentration, the temperature, the electrical conductivity, the amount of floating suspended solids, the turbidity, and the chromaticity of the water system all have a certain correlation with the scaling index value and ion concentrations. In consideration thereof, the water quality information acquiring unit acquires two or more of these water quality parameters to calculate an estimated scaling index value.

While any combination of water quality parameters may be adopted as long as the combination includes two or more of the pH, the cationic demand, the residual chlorine concentration, the temperature, the electrical conductivity, the amount of floating suspended solids, the turbidity, and the chromaticity, the combination preferably includes at least the electrical conductivity. A component that makes up scale is present as ions in water prior to scaling. Since the electrical conductivity is an index which indicates an amount of an ion component in water, the electrical conductivity is particularly useful as an index related to scaling. In addition, the water quality parameters preferably do not include a concentration of cations and a concentration of anions which make up scale. Note that while the residual chlorine concentration is a measurement of the concentration of anions, since instruments for continuous measurement are commercially available, the residual chlorine concentration can be readily measured.

[Relational model information acquiring unit]

The relational model information acquiring unit 21 acquires relational model information including a relational model indicating a relationship between a scaling index value which is calculated from a function created in advance of concentrations of ions constituting scale that may be formed in the water system and the water quality parameters.

The relational model indicates a relationship between a scaling index value which is calculated from a function created in advance of concentrations of ions constituting scale that may be formed in the water system and the water quality parameters. Note that "in advance" means prior to calculating the estimated scaling index value and may be any time prior to calculating the estimated scaling index value including during an operation of calculating the estimated scaling index value or prior to actual operation.

Examples of the relational model include, but are not particularly limited to, a function or a look-up table indicating a relationship between scaling index values and water quality parameters and a trained model representing a relationship between scaling index values and water quality parameters.

It is assumed that the water quality parameters included in the water quality information acquired by the water quality information acquiring unit 20 and the water quality parameters included in the water quality information used in the relational model have two or more water quality parameters that are common to each other among pH, a cationic demand, a residual chlorine concentration, temperature, electrical conductivity, an amount of floating suspended solids, turbidity, and chromaticity.

[Estimating unit]

The estimating unit 22 calculates an estimated scaling index value based on the water quality information and the relational model information.

Specifically, in the estimating unit 22, the estimated scaling index value is calculated by inputting real-time water quality information during operation to the relational model created in advance and by performing a calculation, a collation, or the like.

[Predicting unit]

The predicting unit 23 predicts, based on the estimated scaling index value, a presence or absence or a possibility of generation of scale.

The estimated scaling index value acts as an index of generation of scale in the water system. Therefore, the estimated scaling index value is input to a predictive model prepared in advance to predict the presence or absence or the possibility of generation of scale.

In the case of predicting the presence or absence of generation of scale, for example, the estimated scaling index value or a variation per unit time of the estimated scaling index value may be provided with a threshold, and it can be predicted that scale is to be generated when the estimated scaling index value is larger (or smaller) than the threshold.

In the case of predicting the possibility of generation of scale, for example, a plurality of thresholds may be set to the estimated scaling index value or a variation per unit time of the estimated scaling index value in order to provide a division into three stages, and it can be predicted that: scale will surely occur when the estimated scaling index value or the variation per unit time of the estimated scaling index value is in a first stage; scale may possibly occur when the estimated scaling index value or the variation per unit time of the estimated scaling index value is in a second stage; and scale will surely not occur when the estimated scaling index value or the variation per unit time of the estimated scaling index value is in a third stage. Alternatively, the estimated scaling index value or a variation per unit time of the estimated scaling index value and an occurrence probability can be converted into a function or a learned model from statistical data of actual operation to calculate a probability of scale generation.

[Relational model creating unit]

The relational model creating unit 24 creates a relational model to be acquired by the relational model information acquiring unit 21 and to be used by the estimating unit 22 to calculate an estimated scaling index value.

For example, a relational model is created as follows. Prior to calculating the estimated scaling index value, concentrations of ions that make up scale are measured and, based on the concentrations, a scaling index value is calculated. In addition, in the same water system, two or more water quality parameters selected from the group consisting of pH, a cationic demand, a residual chlorine concentration, temperature, electrical conductivity, an amount of floating suspended solids, turbidity, and chromaticity are measured. A data set of the scaling index value and the water quality parameters are prepared in plurality so that variations arise in the scaling index value and the numerical values of the water quality parameters by, for example, changing a date or time at which measurement is performed. Next, assuming that the scaling index value is a function of water quality parameters (two or more parameters), the function is compared with an actual scaling index value to determine a form and coefficients of the function and a relational model is constructed. In this case, when determining coefficients of the function by a comparison between the function and an actual scaling index value, a regression analysis method (a linear model, a generalized linear model, or a generalized linear mixed model), a decision tree (a decision tree, a regression tree, a random forest, XGBoost, or the like), a neural network (a simple perceptron, a DNN, a CNN, an RNN, or the like), Bayes (naive Bayes or the like), clustering (k-means, k-means ++, or the like), and ensemble learning (Boosting, Adaboost, or the like) can be used.

In an embodiment, the relational model is preferably a model obtained by a regression analysis of the scaling index value and a scaling index estimation function that is a function of the water quality parameters. A specific method of regression analysis will be described later.

When scale is calcium oxalate, the ions that make up the scale are calcium ions and oxalate ions, when scale is calcium carbonate, the ions that make up the scale are calcium ions and carbonate ions, when scale is calcium sulfate, the ions that make up the scale are calcium ions and sulfate ions, when scale is calcium phosphate, the ions that make up the scale are calcium ions and phosphate ions, and when scale is barium sulfate, the ions that make up the scale are barium ions and sulfate ions. Note that the concentration of calcium ions may be replaced with calcium hardness and concentration of carbonate ions may be replaced with m-alkalinity.

The creation of the relational model is preferably performed in the same water system as the water system for which an estimated scaling index value is to be calculated. In addition, for example, in a case where the water quality of a water system changes significantly even in the same device (for example, a case where pulp that is a papermaking raw material is changed or the like in a papermaking system at a paper mill), a relational model with respect to the water system after the water quality changes is preferably created and used.

From such a viewpoint, every time concentrations of ions that make up scale are measured on a regular or irregular basis during operation of the water system, a relational model may be created or a relational model may be updated by adding data.

Note that since the relational model creating unit 24 is not an essential constituent element, the creation of a predictive model may be manually performed by an operator or the like.

[Predictive model creating unit]

The predictive model creating unit 25 creates a predictive model to be acquired by the predictive model information acquiring unit 26 to be described later and to be used by the predicting unit 23 to predict a presence or absence or a possibility of scale generation.

In this case, the predictive model refers to a model indicating a relationship between a scaling index value and a presence or absence of scale generation or a generation amount of scale.

Examples of the predictive model include, but are not particularly limited to, a function or a look-up table indicating a relationship between a scaling index value and a presence or absence of scale generation or a generation amount of scale and a trained model representing a relationship between a scaling index value and a presence or absence of scale generation or a generation amount of scale.

For example, a predictive model is created as follows. Prior to calculating the estimated scaling index value, concentrations of ions that make up scale are measured and, based on the concentrations, a scaling index value is calculated. In addition, in the same water system, a presence or absence of generation of scale is confirmed or a generation amount of scale is measured. A data set of the scaling index value and the presence or absence of scale generation or the generation amount of scale are prepared in plurality so that variations arise in the scaling index value and the numerical values of the presence or absence of scale generation or the generation amount of scale by, for example, changing a date or time at which measurement is performed. Next, a predictive model is constructed by using a probability of scale generation or the generation amount of scale as a function of the scaling index value. Alternatively, the predictive model may be constructed by, for example, after preparing a plurality of data sets of the scaling index value and the presence or absence of scale generation or the generation amount of scale, setting a threshold at a point where scale generation becomes prominent, a point where the scale generation amount equals or exceeds a permissible amount, or the like.

The creation of the predictive model is preferably performed in the same water system as the water system for which an estimated scaling index value is to be calculated. In addition, for example, in a case where the water quality of a water system changes significantly even in the same device (for example, a case where pulp that is a papermaking raw material is changed or the like in a papermaking system at a paper mill), a predictive model with respect to the water system after the water quality changes is preferably created and used.

Note that the predictive model creating unit 25 is not an essential constituent element and the creation of a predictive model may be manually performed by an operator or the like.

[Predictive model information acquiring unit]

The predictive model information acquiring unit 26 acquires a predictive model. The predictive model may be a predictive model created by the predictive model creating unit 25.

[Scale inhibitor addition model creating unit]

The scale inhibitor addition model creating unit 27 creates a scale inhibitor addition model to be acquired by the predictive model information acquiring unit 26 to be described later and to be used by the scale inhibitor adding unit 5 to add a proper amount of a scale inhibitor in accordance with the estimated scaling index value.

In this case, a scale inhibitor addition model refers to a model indicating a relationship between an estimated scaling index value and an addition amount (for example, a minimum addition amount) of a scale inhibitor.

Examples of the scale inhibitor addition model include, but are not particularly limited to, a function or a look-up table indicating a relationship between scaling index values and addition amounts of a scale inhibitor and a trained model representing a relationship between scaling index values and addition amounts of a scale inhibitor.

For example, a scale inhibitor addition model is created as follows. Prior to adding a scale inhibitor, aqueous solutions (pH and temperature of which are constant) containing known concentrations of ions which make up scale (in other words, both a solubility product and a scaling index value are known) are separately prepared for a cation and an anion. After a predetermined amount of the scale addition agent is added to and mixed with the cation aqueous solution, the anion aqueous solution is added to and mixed with the cation aqueous solution. The concentration of any one ion among the cation and the anion of a filtrate obtained by filtering the obtained mixture is measured. A similar operation is performed by changing the addition amount of the scale inhibitor and a smallest addition amount (minimum addition amount) of the scale inhibitor at which a theoretical ion concentration of the mixture and the ion concentration of the filtrate coincide with each other is calculated. The operation described above is repetitively performed a plurality of times by changing concentrations of ions (in other words, by changing the scaling index value) and a relationship between the estimated scaling index value and the addition amount of the scale inhibitor is modeled. Note that a "cation aqueous solution" and an "anion aqueous solution" are aqueous solutions containing a cation and an anion that make up scale and, for example, in the case of calcium oxalate scale, the cation aqueous solution is a calcium chloride solution and the anion aqueous solution is an oxalate solution. As exemplified by chlorine ions contained in the cation aqueous solution, the cation aqueous solution and the anion aqueous solution may contain a counterion of the cation and the anion that make up scale and a type of the counterion is not particularly limited.

The scale inhibitor is not particularly limited as long as formation of object scale can be prevented and examples of scale inhibitors which can be used include a polymer obtained by polymerizing a carboxylic acid-based monomer of acrylic acid, methacrylic acid, maleic acid, itaconic acid, or the like, a copolymer obtained by polymerizing the carboxylic acid-based monomer and a monomer of at least one of styrenesulfonic acid, vinyl alcohol, acrylamide, 2 -hydroxy- 3 - allyloxy- 1 -prop anesulfonic acid, nitrotrimethylene phosphonic acid, hydroxy ethylidene diphosphonic acid, phosphonobutane tricarboxylic acid, sodium tripolyphosphate, and sodium hexametaphosphate.

Note that the scale inhibitor addition model creating unit 27 is not an essential constituent element and the creation of a scale inhibitor addition model may be manually performed by an operator or the like.

[Scale inhibitor addition model information acquiring unit]

The scale inhibitor addition model information acquiring unit 28 acquires a scale inhibitor addition model. The scale inhibitor addition model may be, for example, a scale inhibitor addition model created by the scale inhibitor addition model creating unit 27.

[Additive amount determining unit]

The additive amount determining unit 29 determines an additive amount of a scale inhibitor based on an estimated scaling index value and a scale inhibitor addition model. The estimated scaling index value may be an estimated scaling index value calculated by the estimating unit 22 and the scale inhibitor addition model may be a scale inhibitor addition model created by the scale inhibitor addition model creating unit 27.

A minimum additive amount of the scale inhibitor at a condition of the estimated scaling index value is obtained from the estimated scaling index value and the scale inhibitor addition model. In the additive amount determining unit 29, the minimum additive amount may be adopted as it is as the additive amount or the additive amount may be created by adding a factor of safety or the like to the minimum additive amount.

The additive amount determined at this point is transmitted to the scale inhibitor adding unit 5 to be described later and the scale inhibitor is added to the water system from the scale inhibitor adding unit 5 by the additive amount.

Note that the scale generation amount predicted by the predicting unit 23 may be used instead of the estimated scaling index value calculated by the estimating unit 22.

[Output unit]

The output unit 3 is configured to output any of an estimated scaling index value and a presence or absence or a possibility of generation of scale having been predicted by the predicting unit 23. The estimated scaling index value may be an estimated scaling index value calculated by the estimating unit 22 and the presence or absence or the possibility of generation of scale may be a presence or absence or a possibility of generation of scale predicted by the predicting unit 23.

When the output unit 3 outputs the estimated scaling index value, for example, the estimated scaling index value may be displayed in a chronological manner (such as a graph of estimated scaling index value against time).

When the output unit 3 outputs the presence or absence or the possibility of generation of scale, for example, the output unit 3 may output a warning when the possibility of scale generation exceeds a certain threshold.

Note that the output unit 3 may display water quality parameters and the like in addition to the estimated scaling index value and the presence or absence or the possibility of generation of scale described above and, in particular, the water quality parameters may be displayed in a chronological manner.

[Water quality information measuring unit]

The water quality information measuring unit 4 measures water quality parameters of a water system that is an object for predicting scale generation.

Measurement devices differ depending on contents of water quality parameters to be measured and various sensors or the like can be selected. For example, when measuring pH, a pH meter can be used as the measurement device. When measuring a cationic demand, a colloidal particle charge meter can be used as the measurement device. When measuring residual chlorine concentration, a residual chlorine meter can be used as the measurement device. When measuring temperature, a thermometer can be used as the measurement device. When measuring electrical conductivity, an electrical conductivity meter can be used as the measurement device. When measuring an amount of floating suspended solids, an instrument for measuring a concentration of the floating suspended solids can be used as the measurement device. When measuring turbidity, a turbidimeter can be used as the measurement device. When measuring chromaticity, a colorimeter can be used as the measurement device.

[Scale inhibitor adding unit]

The scale inhibitor adding unit 5 adds a scale inhibitor to the water system in an amount based on an instruction issued by the additive amount determining unit 29 described above.

[Application of prediction system: Bleaching process of kraft pulp at paper mill]

For example, at a paper mill, in addition to oxalate ions and calcium ions eluted in a water system from inside raw material chips of kraft pulp, service water also contains calcium ions and, in particular, since oxalate ions are generated by oxidation of organic substances during a bleaching process, concentrations of oxalate ions and calcium ions tend to reach high levels in the water system during the bleaching process of kraft pulp. In addition, when oxalate ions and calcium ions exceed certain concentrations in this manner, the ions become calcium oxalate and scale is generated.

In this system, as described in NPL1, a saturation index SI of calcium oxalate is represented by equation (5) below, where $K_{sp}$ denotes a solubility product of calcium ions and oxalate ions.

$$SI = log \frac{[Ca^{2+}][C_2O_4^{2-}]}{K_{sp}} \quad \cdots (5)$$

On the other hand, assuming that an estimated saturation index $SI_{est}$ of a water system in a bleaching process of kraft pulp is a function of temperature, pH, and electrical conductivity, the estimated saturation index $SI_{est}$ is represented by equation (6) below, where $b_0$ to $b_8$ denote coefficients. Note that $b_0$ to $b_8$ may be either positive or negative and either integers or decimals, and any six or less among $b_0$ to $b_8$ may be 0 (a minimum of two water quality parameters need be considered), in which case the water quality parameters related to such coefficients need not be measured.

$$Y\{SI_{est}\} = \{b_0 + (b_1 \times pH) + (b_2 \times \text{cationic demand}) + (b_3 \times \text{residual chlorine}$$

$$\text{concentration}) + (b_4 \times \text{temperature}) + (b_5 \times \text{electrical conductivity}) + (b_6 \times$$

$$\text{amount of floating suspended solids}) + (b_7 \times \text{turbidity}) + (b_8 \times$$

$$\text{chromaticity}) \quad \dots (6)$$

$$log \frac{SI_{est}}{1 - SI_{est}}, \frac{1}{SI_{est}}, \frac{1}{SI_{est}^2}$$

where $Y\{SI_{est}\}$ is any of $SI_{est}$, $logSI_{est}$,

Next, in the water system, data sets including a calcium ion concentration, an oxalate ion concentration, pH, a cationic demand, a residual chlorine concentration, temperature, electrical conductivity, an amount of floating suspended solids, turbidity, and chromaticity are measured over time to obtain a plurality of data sets.

Subsequently, using analysis software, the obtained data sets are respectively substituted into SI and $SI_{est}$ to perform regression analysis, and coefficients $b_0$ to $b_8$ are obtained and substituted into equation (6) above to obtain a relational model indicating a relationship between a scaling index value and water quality parameters.

Once such a relational model is obtained, during actual operation, $SI_{est}$ as an approximation of SI can be calculated by measuring only pH, a cationic demand, a residual chlorine concentration, temperature, electrical conductivity, an amount of floating suspended solids, turbidity, and chromaticity. In other words, with the prediction system (estimation system) according to the present embodiment, a calcium ion concentration or an oxalate ion concentration need not necessarily be measured in order to assess the easiness of generation of scale and a saturation index can be obtained in an instantaneous and continuous manner.

In this system, it is predicted that the larger the obtained value of $SI_{est}$, the more likely that scale is to be generated and, accordingly, a warning is displayed on the output unit 3. An operator viewing the warning can add a scale inhibitor to the water system in accordance with a severity of the warning. Accordingly, inhibition of scale can be performed and, at the same time, scale inhibition which is normally added when scale is not generated can also be reduced.

While a specific example of a case of a water system being a bleaching process of kraft pulp at a paper mill has been described above, when other water systems are to be evaluated, equation (5) can be used as it is by changing the species of ions that constitute scale when necessary.

In addition, while a bleaching process at a paper mill has been described above as a water system, water systems to be an object of the prediction system and the like according to the present embodiment are not particularly limited and, in the case of a paper mill, the water system may be a cooking process, a washing process, a black liquor-concentration process, a caustification process, and the like. Furthermore, besides water systems other than those of paper mills, water systems to be an object include water systems at, for example, an ironworks such as: a blast furnace dust collection water system; a converter dust collection water system; a direct cooling water system and an indirect cooling water system of a continuous casting process, a hot rolling process, or a cold rolling process; and a water system of leachates or drainage originating inside the premises. Moreover, water systems to be an object include a desulfurization facility, a drainage water system, a dust collection water system, and the like at a power plant or a coal chemical plant. In addition, water systems to be an object include various piping, heat exchangers, storage tanks, kilns, and cleaning equipment other than the water systems exemplified above.

[Hardware configuration of prediction system]

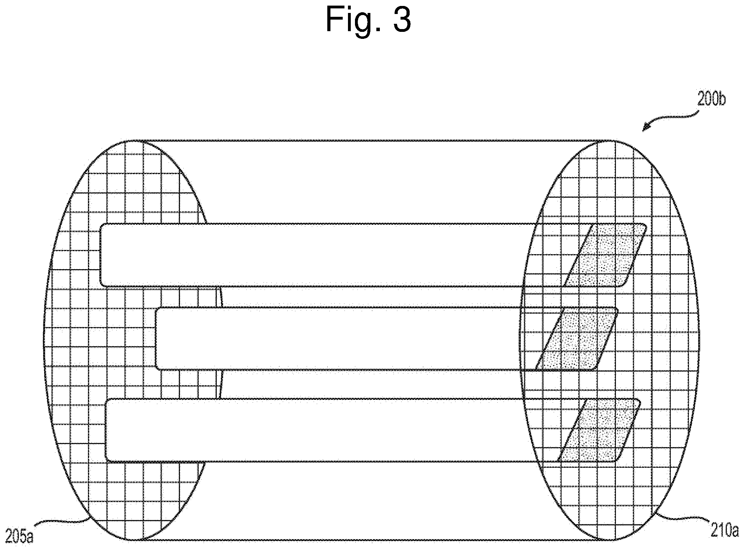

Fig. 3 is a schematic diagram showing a hardware configuration of the prediction device according to the present embodiment. As shown in Fig. 3, the prediction device 2 includes a communicating unit 61, a storage unit 62, and a control unit 63, and these constituent elements are electrically connected via a communication bus 64 inside the prediction device 2. Hereinafter, the constituent elements will be further described.

While the communicating unit 61 is preferably wired communication means such as USB, IEEE 1394, Thunderbolt, and wired LAN network communication, the communicating unit 61 can include wireless LAN network communication, mobile communication such as 3G/LTE/5G, Bluetooth (registered trademark) communication, and the like when necessary. In other words, more preferably, the communicating unit 61 is implemented as an assembly of the plurality of communication means described above. Accordingly, exchange of information and commands is executed between the prediction device 2 and other devices capable of communicating with the prediction device 2.

The storage unit 62 stores various information defined according to the description provided above. The storage unit 62 can be implemented as, for example, a storage device such as a solid state drive (SSD) or a memory such as a random access memory (RAM) which stores information (arguments, arrays, and the like) temporarily necessary in relation to calculations of a program. In addition, the storage unit 62 may be a combination thereof. Furthermore, the storage unit 62 stores various programs which can be read by the control unit 63 to be described later.

The control unit 63 performs processing and control of overall operations related to the prediction device 2. The control unit 63 is, for example, a central processing unit (CPU, not illustrated). The control unit 63 realizes various functions related to the prediction device 2 by reading a predetermined program stored in the storage unit 62. In other words, by causing information processing by software (stored in the storage unit 62) to be concretely realized by hardware (the control unit 63), the information processing can be executed as respective functional units in the control unit 63 as shown in Fig. 3. While a single control unit 63 is notated in Fig. 3, actual configurations are not limited thereto and a configuration may be adopted in which a plurality of control units 63 are provided for each function or a single control unit and a plurality of control units may be combined with each other.

<Estimation system>

An estimation system according to the present embodiment is an estimation system which estimates a scaling index value of a water system. Specifically, the estimation system includes: a water quality information acquiring unit; a relational model information acquiring unit; and an estimating unit. The water quality information acquiring unit acquires water quality information including two or more water quality parameters selected from the group consisting of at least electrical conductivity, pH, a cationic demand, a residual chlorine concentration, temperature, an amount of floating suspended solids, turbidity, and chromaticity of the water system. The relational model information acquiring unit acquires relational model information including a relational model indicating a relationship between a scaling index value which is calculated from a function created in advance of concentrations of ions constituting scale that may be formed in the water system and the water quality parameters. The estimating unit calculates an estimated scaling index value from the water quality information and the relational model.

Note that as the water quality information acquiring unit, the relational model information acquiring unit, and the estimating unit, since units similar to those of the prediction system described above can be used, a description thereof will be omitted.

<Control system>

A control system according to the present embodiment is a control system which controls addition of a scale inhibitor with respect to a water system. Specifically, the control system includes a water quality information acquiring unit, a relational model information acquiring unit, an estimating unit, a scale inhibitor addition model information acquiring unit, and an additive amount determining unit. Among these units, the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system. The relational model information acquiring unit acquires relational model information including a relational model indicating a relationship between a scaling index value which is calculated from a function created in advance of concentrations of ions constituting scale that may be formed in the water system and the water quality parameters. The estimating unit calculates an estimated scaling index value based on the water quality information and the relational model information. The scale inhibitor addition model information acquiring unit acquires scale inhibitor addition model information including a scale inhibitor addition model which has been created in advance and which indicates a relationship between the estimated scaling index value and an additive amount of a scale inhibitor. The additive amount determining unit determines an additive amount of the scale inhibitor based on the estimated scaling index value and the scale inhibitor addition model information.

Note that in the control system, particularly when the control system includes the scale inhibitor adding unit, the control system may be used as an addition system which adds a scale inhibitor with respect to a water system.

<Estimation device>

An estimation device according to the present embodiment is an estimation device which estimates a scaling index value of a water system. Specifically, the estimation device includes: a water quality information acquiring unit; a relational model information acquiring unit; and an estimating unit. Among these units, the water quality information acquiring unit acquires water quality information including two or more water quality parameters selected from the group consisting of at least electrical conductivity, pH, a cationic demand, a residual chlorine concentration, temperature, an amount of floating suspended solids, turbidity, and chromaticity of the water system. The relational model information acquiring unit acquires relational model information including a relational model indicating a relationship between a scaling index value which is calculated from a function created in advance of concentrations of ions constituting scale that may be formed in the water system and the water quality parameters. The estimating unit calculates an estimated scaling index value from the water quality information and the relational model.

Note that as the water quality information acquiring unit, the relational model information acquiring unit, and the estimating unit, since units similar to those of the prediction system described above can be used, a description thereof will be omitted.

<Control device>

A control device according to the present embodiment is a control device which controls addition of a scale inhibitor with respect to a water system. Specifically, the control device includes a water quality information acquiring unit, a relational model information acquiring unit, an estimating unit, a scale inhibitor addition model information acquiring unit, and an additive amount determining unit. Among these units, the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system. The relational model information acquiring unit acquires relational model information including a relational model indicating a relationship between a scaling index value which is calculated from a function created in advance of concentrations of ions constituting scale that may be formed in the water system and the water quality parameters. The estimating unit calculates an estimated scaling index value based on the water quality information and the relational model information. The scale inhibitor addition model information acquiring unit acquires scale inhibitor addition model information including a scale inhibitor addition model which has been created in advance and which indicates a relationship between the estimated scaling index value and an additive amount of a scale inhibitor. The additive amount determining unit determines an additive amount of the scale inhibitor based on the estimated scaling index value and the scale inhibitor addition model information.

Note that as the water quality information acquiring unit, the relational model information acquiring unit, the estimating unit, the scale inhibitor addition model information acquiring unit, and the additive amount determining unit, since units similar to those of the prediction system described above can be used, a description thereof will be omitted.

<Prediction program>

A prediction program according to the present embodiment is a prediction program for predicting scale generation in a water system. Specifically, the prediction program causes a computer to function as: a water quality information acquiring unit; a relational model information acquiring unit; an estimating unit; and a predicting unit. Among these units, the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system. The relational model information acquiring unit acquires relational model information including a relational model indicating a relationship between a scaling index value which is calculated from a function created in advance of concentrations of ions constituting scale that may be formed in the water system and the water quality parameters. The estimating unit calculates an estimated scaling index value based on the water quality information and the relational model information. The predicting unit predicts, based on the estimated scaling index value, a presence or absence or a possibility of generation of scale.

Note that as the water quality information acquiring unit, the relational model information acquiring unit, the estimating unit, and the predicting unit, since units similar to those of the prediction system described above can be used, a description thereof will be omitted.

<Estimation program>

An estimation program according to the present embodiment is an estimation program for estimating a scaling index value of a water system. Specifically, the estimation program causes a computer to function as: a water quality information acquiring unit; a relational model information acquiring unit; and an estimating unit. Among these units, the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system. The relational model information acquiring unit acquires relational model information including a relational model indicating a relationship between a scaling index value which is calculated from a function created in advance of concentrations of ions constituting scale that may be formed in the water system and the water quality parameters. The estimating unit calculates an estimated scaling index value based on the water quality information and the relational model information.

Note that as the water quality information acquiring unit, the relational model information acquiring unit, and the estimating unit, since units similar to those of the prediction system described above can be used, a description thereof will be omitted.

<Control program>

A control program according to the present embodiment is a control program for controlling addition of a scale inhibitor with respect to a water system. Specifically, the control program causes a computer to function as: a water quality information acquiring unit; a relational model information acquiring unit; an estimating unit; a scale inhibitor addition model information acquiring unit; and an additive amount determining unit. Among these units, the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system. The relational model information acquiring unit acquires relational model information including a relational model indicating a relationship between a scaling index value which is calculated from a function created in advance of concentrations of ions constituting scale that may be formed in the water system and the water quality parameters. The estimating unit calculates an estimated scaling index value based on the water quality information and the relational model information. The scale inhibitor addition model information acquiring unit acquires scale inhibitor addition model information including a scale inhibitor addition model which has been created in advance and which indicates a relationship between the estimated scaling index value and an additive amount of a scale inhibitor. The additive amount determining unit determines an additive amount of the scale inhibitor based on the estimated scaling index value and the scale inhibitor addition model information.

Note that as the water quality information acquiring unit, the relational model information acquiring unit, the estimating unit, the scale inhibitor addition model information acquiring unit, and the additive amount determining unit, since units similar to those of the prediction system described above can be used, a description thereof will be omitted.

Even when not described as essential components in the estimation system, the control system, the addition system, the estimation device, the control device, the prediction program, the estimation program, and the control program described above, the predicting unit, the relational model creating unit, the predictive model creating unit, the predictive model information acquiring unit, the scale inhibitor addition model creating unit, the scale inhibitor addition model information acquiring unit, and the additive amount determining unit can be provided, and since units similar to those of the prediction system described above can be used, a description thereof will be omitted. In addition, even when not described as essential components in the estimation system, the control system, and the addition system described above, the output unit, the water quality information measuring unit, and the scale inhibitor adding unit can be provided, and since units similar to those of the prediction system described above can be used, a description thereof will be omitted.

<Prediction method>

A prediction method according to the present embodiment is a prediction method of predicting scale generation in a water system. Specifically, the prediction method includes: a water quality information acquiring step; a relational model information acquiring step; an estimating step; and a predicting step. Among these steps, in the water quality information acquiring step, water quality information including two or more water quality parameters selected from the group consisting of at least pH, a cationic demand, a residual chlorine concentration, temperature, electrical conductivity, an amount of floating suspended solids, turbidity, and chromaticity of the water system is acquired. In the relational model information acquiring step, relational model information including a relational model indicating a relationship between a scaling index value which is calculated from a function created in advance of concentrations of ions constituting scale that may be formed in the water system and the water quality parameters is acquired. In the estimating step, an estimated scaling index value is calculated based on the water quality information and the relational model information. In the predicting step, a presence or absence or a possibility of generation of scale is predicted based on the estimated scaling index value.

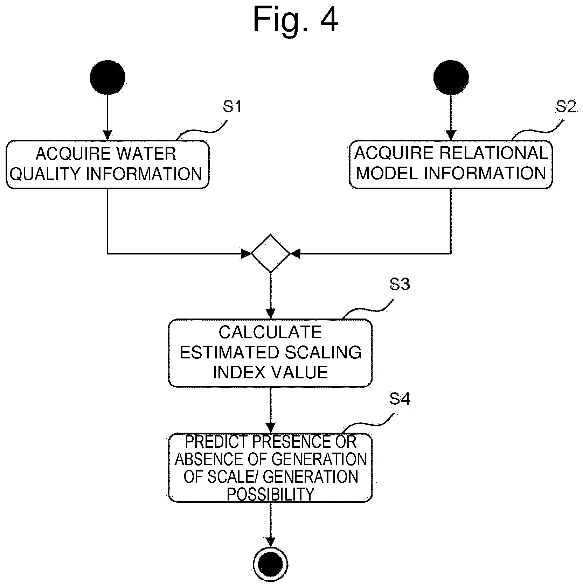

Fig. 4 is a flowchart of a prediction method according to the present embodiment. As shown in Fig. 4, in a support method according to the present embodiment, water quality information is acquired (water quality information acquiring step S1), relational model information is acquired (relational model information acquiring step S2), and with the water quality information and the relational model information as input information, an estimated scaling index value is calculated (estimating step S3). Next, with the estimated scaling index value as input information, a presence or absence or a possibility of generation of scale is predicted (predicting step S4).

<Estimation method>

An estimation method according to the present embodiment is an estimation method of estimating a scaling index value of a water system. Specifically, the estimation method includes: a water quality information acquiring step; a relational model information acquiring step; and an estimating step. Among these steps, in the water quality information acquiring step, water quality information including two or more water quality parameters selected from the group consisting of at least pH, a cationic demand, a residual chlorine concentration, temperature, electrical conductivity, an amount of floating suspended solids, turbidity, and chromaticity of the water system is acquired. In the relational model information acquiring step, relational model information including a relational model indicating a relationship between a scaling index value which is calculated from a function created in advance of concentrations of ions constituting scale that may be formed in the water system and the water quality parameters is acquired. In the estimating step, an estimated scaling index value is calculated based on the water quality information and the relational model information.

Since a flowchart of the estimation method is similar to up to the estimating step S3 in the flowchart shown in Fig. 4, a description will be omitted.

<Control method>

A control method according to the present embodiment is a control method of controlling addition of a scale inhibitor with respect to a water system. Specifically, the control method includes: a water quality information acquiring step; a relational model information acquiring step; an estimating step; a scale inhibitor addition model information acquiring step; and an additive amount determining step. Among these steps, in the water quality information acquiring step, water quality information including two or more water quality parameters selected from the group consisting of at least pH, a cationic demand, a residual chlorine concentration, temperature, electrical conductivity, an amount of floating suspended solids, turbidity, and chromaticity of the water system is acquired. In the relational model information acquiring step, relational model information including a relational model indicating a relationship between a scaling index value which is calculated from a function created in advance of concentrations of ions constituting scale that may be formed in the water system and the water quality parameters is acquired. In the estimating step, an estimated scaling index value is calculated based on the water quality information and the relational model information. In the scale inhibitor addition model information acquiring step, scale inhibitor addition model information including a scale inhibitor addition model which has been created in advance and which indicates a relationship between the estimated scaling index value and an additive amount of a scale inhibitor is acquired. In the additive amount determining step, an additive amount of the scale inhibitor is determined based on the estimated scaling index value and the scale inhibitor addition model information.

Note that since the water quality information acquiring step, the relational model information acquiring step, and the estimating step in the prediction method, the estimation method, and the control method are similar to operations of the water quality information acquiring unit, the relational model information acquiring unit, and the estimating unit, a description thereof will be omitted.

Even when not described as essential components in the prediction method, the estimation method, and the control method described above, the predicting step, a relational model creating step, a predictive model creating step, a predictive model information acquiring step, a scale inhibitor addition model creating step, the scale inhibitor addition model information acquiring step, and the additive amount determining step can be provided, and since the steps are similar to operations of the predicting unit, the relational model creating unit, the predictive model creating unit, the predictive model information acquiring unit, the scale inhibitor addition model creating unit, the scale inhibitor addition model information acquiring unit, and the additive amount determining unit, a description thereof will be omitted.

Examples

While the present invention will be described below in more concrete terms by showing examples, it is to be understood that the following examples are not intended to limit the present invention in any way whatsoever.

Example 1

In a chlorine dioxide bleaching tower at a paper mill A where calcium oxalate scale is generated, a data set including pH, electrical conductivity, temperature, oxalate ion concentration, and calcium ion concentration of washing water was measured 19 times on different dates and at different times. With respect to all of the data sets, a scaling index value (SI) was obtained using equation (1) from the oxalate ion concentration and the calcium ion concentration.

Next, with respect to 10 data sets randomly selected from the total of 19 data sets, a regression analysis of a relationship between the scaling index value and the pH, the electrical conductivity, and the temperature was performed using an analysis tool of Microsoft Office Excel manufactured by Microsoft Corporation. As a result, a relational model having a correlation coefficient of 0.919 ($p < 0.05$) and using pH, electrical conductivity, and temperature as explanatory variables was obtained.

Subsequently, with respect to the remaining nine data sets among the total of 19 data sets, estimated scaling index values were calculated using the relational model and pH, electrical conductivity, and temperature. The correlation coefficient of the nine data sets was 0.831 ($p < 0.05$).

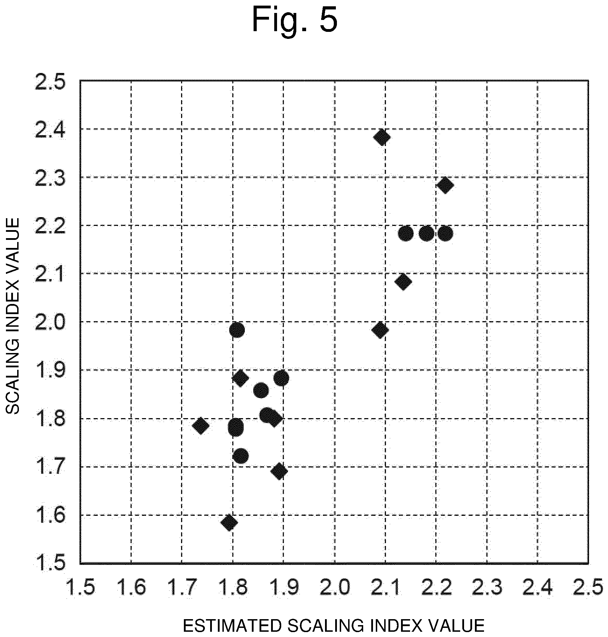

Fig. 5 shows plots of scaling index values against estimated scaling index values of the total of 19 data sets according to example 1. In Fig. 5, an axis of abscissa represents the estimated scaling index value obtained by regression analysis and an axis of ordinate represents the scaling index value obtained from the temperature, the oxalate ion concentration, and the calcium ion concentration.

As described above, it was found that using pH, electrical conductivity, temperature, and a relational model enables an estimated scaling index value which is correlated with a scaling index value to be calculated without having to use an oxalate ion concentration and a calcium ion concentration.

Example 2

In circulating water of a flue gas desulfurization facility at a plant B where calcium carbonate scale is generated, a data set including pH, electrical conductivity, temperature, turbidity, m-alkalinity, and calcium hardness of the circulating water was measured 18 times on different dates and at different times. With respect to all of the data sets, a scaling index value (SI) was obtained using measurement results of m-alkalinity (corresponding to carbonate ion concentration) and calcium hardness (corresponding to calcium ion concentration).

Next, with respect to 11 data sets randomly selected from the total of 18 data sets, a regression analysis of a relationship between the scaling index value and the pH, the electrical conductivity, the temperature, and the turbidity was performed using an analysis tool of Microsoft Office Excel manufactured by Microsoft Corporation. As a result, a relational model having a correlation coefficient of 0.926 ($p < 0.001$) and using pH, electrical conductivity, temperature, and turbidity as explanatory variables was obtained.

Subsequently, with respect to the remaining seven data sets among the total of 11 data sets, estimated scaling index values were calculated using the relational model and pH, electrical conductivity, temperature, and turbidity. The correlation coefficient of the three data sets was 0.976 ($p < 0.001$).

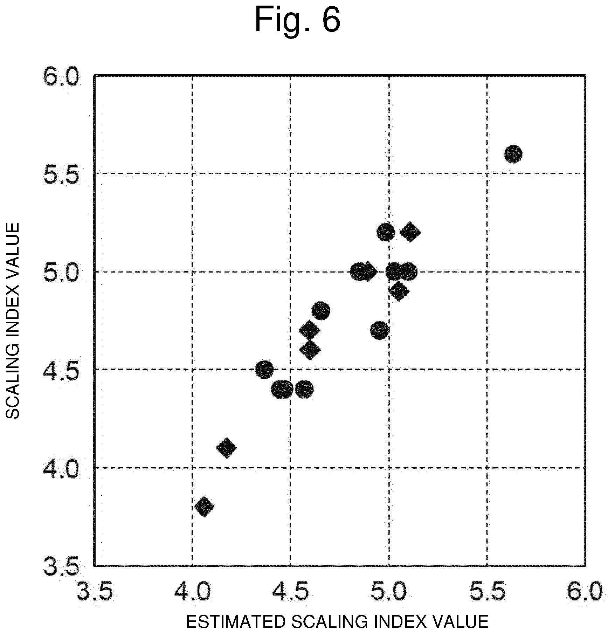

Fig. 6 shows plots of scaling index values against estimated scaling index values of the total of 18 data sets according to example 2. In Fig. 6, an axis of abscissa represents the estimated scaling index value obtained by regression analysis and an axis of ordinate represents the scaling index value obtained from the temperature, the pH, the m-alkalinity, and the calcium hardness.

As described above, it was found that using pH, electrical conductivity, temperature, turbidity, and a relational model enables an estimated scaling index value which is correlated with a scaling index value to be calculated without having to use a carbonate ion concentration and a calcium ion concentration.

Reference Signs List

1 prediction system
2 prediction device
3 output unit or output device
4 water quality information measuring unit or water quality information measurement device
5 scale inhibitor adding unit or scale inhibitor addition device
20 water quality information acquiring unit
21 relational model information acquiring unit
22 estimating unit
23 predicting unit
24 relational model creating unit
25 predictive model creating unit
26 predictive model information acquiring unit
27 scale inhibitor addition model creating unit
28 scale inhibitor addition model information acquiring unit
29 additive amount determining unit
61 communicating unit
62 storage unit
63 control unit
64 communication bus

**Claims**

1. An estimation device which estimates a scaling index value of a water system, the estimation device comprising:

   a water quality information acquiring unit; a relational model information acquiring unit; and an estimating unit, wherein
   the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system,

the relational model information acquiring unit acquires relational model information including a relational model indicating a relationship between a scaling index value which is calculated from a function created in advance of concentrations of ions constituting scale that may be formed in the water system and the water quality parameters, and

the estimating unit calculates an estimated scaling index value based on the water quality information and the relational model information.

2. The estimation device according to claim 1, wherein
the relational model is a model obtained by a regression analysis of the scaling index value and a scaling index estimation function that is a function of the water quality parameters.

3. The estimation device according to claim 1 or 2, wherein
the water quality parameters include at least electrical conductivity.

4. A prediction device which predicts scale generation in a water system, the prediction device comprising:

a water quality information acquiring unit; a relational model information acquiring unit; an estimating unit; and a predicting unit, wherein
the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system,
the relational model information acquiring unit acquires relational model information including a relational model indicating a relationship between a scaling index value which is calculated from a function created in advance of concentrations of ions constituting scale that may be formed in the water system and the water quality parameters,
the estimating unit calculates an estimated scaling index value based on the water quality information and the relational model information, and
the predicting unit predicts, based on the estimated scaling index value, a presence or absence or a possibility of generation of scale.

5. A control device which controls addition of a scale inhibitor with respect to a water system, the control device comprising:

a water quality information acquiring unit; a relational model information acquiring unit; an estimating unit; a scale inhibitor addition model information acquiring unit; and an additive amount determining unit, wherein
the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system,
the relational model information acquiring unit acquires relational model information including a relational model indicating a relationship between a scaling index value which is calculated from a function created in advance of concentrations of ions constituting scale that may be formed in the water system and the water quality parameters,
the estimating unit calculates an estimated scaling index value based on the water quality information and the relational model information,
the scale inhibitor addition model information acquiring unit acquires scale inhibitor addition model information including a scale inhibitor addition model which has been created in advance and which indicates a relationship between the estimated scaling index value and an additive amount of a scale inhibitor, and
the additive amount determining unit determines an additive amount of the scale inhibitor based on the estimated scaling index value and the scale inhibitor addition model information.

6. An estimation system which estimates a scaling index value of a water system, the estimation system comprising:

a water quality information acquiring unit; a relational model information acquiring unit; and an estimating unit, wherein
the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system,

the relational model information acquiring unit acquires relational model information including a relational model indicating a relationship between a scaling index value which is calculated from a function created in advance of concentrations of ions constituting scale that may be formed in the water system and the water quality parameters, and

the estimating unit calculates an estimated scaling index value based on the water quality information and the relational model information.

7. A prediction system which predicts scale generation in a water system, the prediction system comprising:

a water quality information acquiring unit; a relational model information acquiring unit; an estimating unit; and a predicting unit, wherein

the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system,

the relational model information acquiring unit acquires relational model information including a relational model indicating a relationship between a scaling index value which is calculated from a function created in advance of concentrations of ions constituting scale that may be formed in the water system and the water quality parameters,

the estimating unit calculates an estimated scaling index value based on the water quality information and the relational model information, and

the predicting unit predicts, based on the estimated scaling index value, a presence or absence or a possibility of generation of scale.

8. A control system which controls addition of a scale inhibitor with respect to a water system, the control system comprising:

a water quality information acquiring unit; a relational model information acquiring unit; an estimating unit; a scale inhibitor addition model information acquiring unit; and an additive amount determining unit, wherein

the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system,

the relational model information acquiring unit acquires relational model information including a relational model indicating a relationship between a scaling index value which is calculated from a function created in advance of concentrations of ions constituting scale that may be formed in the water system and the water quality parameters,

the estimating unit calculates an estimated scaling index value based on the water quality information and the relational model information,

the scale inhibitor addition model information acquiring unit acquires scale inhibitor addition model information including a scale inhibitor addition model which has been created in advance and which indicates a relationship between the estimated scaling index value and an additive amount of a scale inhibitor, and

the additive amount determining unit determines an additive amount of the scale inhibitor based on the estimated scaling index value and the scale inhibitor addition model information.

9. An estimation program for estimating a scaling index value of a water system,

the estimation program causing a computer to function as: a water quality information acquiring unit; a relational model information acquiring unit; and an estimating unit, wherein

the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system,

the relational model information acquiring unit acquires relational model information including a relational model indicating a relationship between a scaling index value which is calculated from a function created in advance of concentrations of ions constituting scale that may be formed in the water system and the water quality parameters, and
the estimating unit calculates an estimated scaling index value based on the water quality information and the relational model information.

10. A prediction program for predicting scale generation in a water system,

the prediction program causing a computer to function as: a water quality information acquiring unit; a relational model information acquiring unit; an estimating unit; and a predicting unit, wherein
the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system,
the relational model information acquiring unit acquires relational model information including a relational model indicating a relationship between a scaling index value which is calculated from a function created in advance of concentrations of ions constituting scale that may be formed in the water system and the water quality parameters,
the estimating unit calculates an estimated scaling index value based on the water quality information and the relational model information, and
the predicting unit predicts, based on the estimated scaling index value, a presence or absence or a possibility of generation of scale.

11. A control program for controlling addition of a scale inhibitor with respect to a water system,

the control program causing a computer to function as: a water quality information acquiring unit; a relational model information acquiring unit; an estimating unit; a scale inhibitor addition model information acquiring unit; and an additive amount determining unit, wherein
the water quality information acquiring unit acquires water quality information including two or more water quality parameters selected from the group consisting of at least pH, a cationic demand, a residual chlorine concentration, temperature, electrical conductivity, an amount of floating suspended solids, turbidity, and chromaticity of the water system,
the relational model information acquiring unit acquires relational model information including a relational model indicating a relationship between a scaling index value which is calculated from a function created in advance of concentrations of ions constituting scale that may be formed in the water system and the water quality parameters,
the estimating unit calculates an estimated scaling index value based on the water quality information and the relational model information,
the scale inhibitor addition model information acquiring unit acquires scale inhibitor addition model information including a scale inhibitor addition model which has been created in advance and which indicates a relationship between the estimated scaling index value and an additive amount of a scale inhibitor, and
the additive amount determining unit determines an additive amount of the scale inhibitor based on the estimated scaling index value and the scale inhibitor addition model information.

12. An estimation method of a scaling index value of a water system,

the estimation method comprising: a water quality information acquiring step; a relational model information acquiring step; and an estimating step, wherein
in the water quality information acquiring step, water quality information including two or more water quality parameters selected from the group consisting of at least pH, a cationic demand, a residual chlorine concentration, temperature, electrical conductivity, an amount of floating suspended solids, turbidity, and chromaticity of the water system is acquired,
in the relational model information acquiring step, relational model information including a relational model indicating a relationship between a scaling index value which is calculated from a function created in advance of concentrations of ions constituting scale that may be formed in the water system and the water quality parameters is acquired, and
in the estimating step, an estimated scaling index value is calculated based on the water quality information and the relational model information.

13. A prediction method of scale generation in a water system,

the prediction method comprising: a water quality information acquiring step; a relational model information acquiring step; an estimating step; and a predicting step, wherein

in the water quality information acquiring step, water quality information including two or more water quality parameters selected from the group consisting of at least pH, a cationic demand, a residual chlorine concentration, temperature, electrical conductivity, an amount of floating suspended solids, turbidity, and chromaticity of the water system is acquired,

in the relational model information acquiring step, relational model information including a relational model indicating a relationship between a scaling index value which is calculated from a function created in advance of concentrations of ions constituting scale that may be formed in the water system and the water quality parameters is acquired,

in the estimating step, an estimated scaling index value is calculated based on the water quality information and the relational model information, and

in the predicting step, a presence or absence or a possibility of generation of scale is predicted based on the estimated scaling index value.

14. A control method of addition of a scale inhibitor with respect to a water system,

the control method comprising: a water quality information acquiring step; a relational model information acquiring step; an estimating step; a scale inhibitor addition model information acquiring step; and an additive amount determining step, wherein

in the water quality information acquiring step, water quality information including two or more water quality parameters selected from the group consisting of at least pH, a cationic demand, a residual chlorine concentration, temperature, electrical conductivity, an amount of floating suspended solids, turbidity, and chromaticity of the water system is acquired,

in the relational model information acquiring step, relational model information including a relational model indicating a relationship between a scaling index value which is calculated from a function created in advance of concentrations of ions constituting scale that may be formed in the water system and the water quality parameters is acquired,

in the estimating step, an estimated scaling index value is calculated based on the water quality information and the relational model information, and

in the scale inhibitor addition model information acquiring step, scale inhibitor addition model information including a scale inhibitor addition model which has been created in advance and which indicates a relationship between the estimated scaling index value and an additive amount of a scale inhibitor is acquired, and

in the additive amount determining step, an additive amount of the scale inhibitor is determined based on the estimated scaling index value and the scale inhibitor addition model information.

# Fig. 1

EP 4 273 103 A1

# Fig. 2

EP 4 273 103 A1

Fig. 3

# Fig. 4

ACQUIRE WATER
QUALITY INFORMATION — S1

ACQUIRE RELATIONAL
MODEL INFORMATION — S2

CALCULATE
ESTIMATED SCALING
INDEX VALUE — S3

PREDICT PRESENCE OR
ABSENCE OF GENERATION
OF SCALE/ GENERATION
POSSIBILITY — S4

# Fig. 5

# Fig. 6

## INTERNATIONAL SEARCH REPORT

International application No.

**PCT/JP2022/002303**

### A. CLASSIFICATION OF SUBJECT MATTER

*C02F 5/00*(2006.01)i; *G05B 23/02*(2006.01)i; *C02F 1/00*(2006.01)i; *G06Q 50/06*(2012.01)i; *G01N 33/18*(2006.01)i
FI: C02F5/00 610G; C02F5/00 620B; C02F1/00 D; G05B23/02 R; G01N33/18 C; G06Q50/06

According to International Patent Classification (IPC) or to both national classification and IPC

### B. FIELDS SEARCHED

Minimum documentation searched (classification system followed by classification symbols)

C02F5/00; G05B23/02; C02F1/00; G06Q50/06; G01N33/18

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

Japio-GPG/FX

### C. DOCUMENTS CONSIDERED TO BE RELEVANT

| Category* | Citation of document, with indication, where appropriate, of the relevant passages | Relevant to claim No. |
|---|---|---|
| Y | WO 2015/075835 A1 (KURITA WATER INDUSTRIES LTD.) 28 May 2015 (2015-05-28) paragraphs [0001]-[0062], fig. 1-4 | 1-14 |
| Y | JP 2015-205237 A (KURITA WATER INDUSTRIES LTD.) 19 November 2015 (2015-11-19) paragraphs [0001]-[0113], fig. 1-8 | 1-14 |
| A | JP 2017-140595 A (TOSHIBA CORP.) 17 August 2017 (2017-08-17) claims 1-7 | 1-14 |
| A | WO 2020/021687 A1 (MITSUBISHI ELECTRIC CORP.) 30 January 2020 (2020-01-30) claims 1-16 | 1-14 |
| A | CN 111242381 A (PETRO-CYBERWORKS INFORMATION TECHNOLOGY CO., LTD.) 05 June 2020 (2020-06-05) claims 1-10 | 1-14 |

☐ Further documents are listed in the continuation of Box C. ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **02 March 2022** | **15 March 2022** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 **Japan** | |
| | Telephone No. |

Form PCT/ISA/210 (second sheet) (January 2015)

**INTERNATIONAL SEARCH REPORT**
Information on patent family members

International application No.

**PCT/JP2022/002303**

| Patent document cited in search report | | | Publication date (day/month/year) | Patent family member(s) | | | Publication date (day/month/year) |
|---|---|---|---|---|---|---|---|
| WO | 2015/075835 | A1 | 28 May 2015 | US paragraphs [0001]-[0102], fig. 1-4 CN | 2016/0362305 105764856 | A1 A | |
| JP | 2015-205237 | A | 19 November 2015 | WO paragraphs [0001]-[0113], fig. 1-8 | 2015/159711 | A1 | |
| JP | 2017-140595 | A | 17 August 2017 | (Family: none) | | | |
| WO | 2020/021687 | A1 | 30 January 2020 | US claims 1-16 | 2021/0171383 | A1 | |
| CN | 111242381 | A | 05 June 2020 | (Family: none) | | | |

Form PCT/ISA/210 (patent family annex) (January 2015)

**REFERENCES CITED IN THE DESCRIPTION**

**Non-patent literature cited in the description**

- **KENJI KOWATA.** *JAPAN TAPPI JOURNAL,* 2003, vol. 57 (7), 70

- **KENJI KOWATA.** *Japanese Journal of Paper Technology,* 2001, vol. 44 (7), 29